# EUROPEAN PATENT APPLICATION

(11) **EP 2 671 512 A1**
(43) Date of publication of application: **11.12.2013**
(21) Application number: 12742632.8
(22) Date of filing: 01.02.2012
(51) Int. Cl.: A61B 8/12

(54) **BODY CAVITY ULTRASONIC PROBE**

(30) Priority: 01.02.2011 JP 2011019474
(71) Applicant: Panasonic Corporation, Osaka 571-8501 (JP)
(72) Inventor: HIRAYAMA, Michiyo, Osaka 540-6207 (JP); TAKANO, Hiroshi, Osaka 540-6207 (JP)
(74) Representative: Grünecker, Kinkeldey, Stockmair & Schwanhäusser
(86) International application number: PCT/JP2012/000688
(87) International publication number: WO 2012/105256

(57) **Abstract**

An intracavitary ultrasound probe of the present invention includes: a housing including: an insertion section having a center axis; and a holding section extending in a longitudinal direction of the housing; and an ultrasound unit built into the insertion section. The holding section includes: a first axis that matches with the center axis of the insertion section; and a second axis that passes through a center of a cross section taken in the vicinity of a longitudinal center of the housing, and extends in parallel to the longitudinal direction. In a cross section perpendicular to the longitudinal direction of the holding section, the first axis is offset from the second axis in an offset direction. In the cross section perpendicular to the longitudinal direction of the holding section, the outer surface of the holding section includes: a first outer surface portion located on a line extending from the second axis in the offset direction; and a second outer surface portion located on a line extending from the second axis in a direction opposite to the offset direction. A curvature of the first outer surface portion is smaller than a curvature of the second outer surface portion.

## Description

### TECHNICAL FIELD

The present invention relates to an intracavitary ultrasound probe to be used, for example, to check a growth state of a fetus and a uterine fibroid state.

### BACKGROUND ART

As disclosed in, for example, Patent Document No. 1, a conventional intracavitary ultrasound probe includes an insertion section that includes an ultrasound unit built inside at a position close to a distal end thereof, and a holding section that has a circular cross section and is connected to a rear end of the insertion section. Patent Document No. 1 also discloses a puncture adapter to be mounted to such an intracavitary ultrasound probe as described above.

A doctor grips the holding section, and inserts, in this state, the insertion section into a body cavity to make diagnoses. A reception echo is acquired through transmission and reception of an ultrasonic wave by the intracavitary ultrasound probe, and based on the reception echo, a tomographic image is generated and characteristic values of body tissue and the like are obtained. In this manner, the doctor checks, for example, the growth state of a fetus and the uterine fibroid state as described above.

### CITATION LIST

### PATENT LITERATURE

Patent Document No. 1: Japanese Patent Application Laid-Open Publication No. 2006-288773

### SUMMARY OF INVENTION

### TECHNICAL PROBLEM

According to a study conducted by the inventors of the present application, however, the conventional intracavitary ultrasound probe has a problem of poor operability. In particular, the operability of the intracavitary ultrasound probe may be poor when the intracavitary ultrasound probe is rotated about its axis to obtain diagnosis images of different regions. The present invention has been made to solve such a problem inherent in the conventional technology, and it is therefore an object thereof to provide an intracavitary ultrasound probe having excellent operability.

### SOLUTION TO PROBLEM

According to the present invention, there is provided an intracavitary ultrasound probe, including: a housing including: an insertion section having a center axis; and a holding section extending in a longitudinal direction of the housing, the holding section being connected to one end of the insertion section so that the center axis and the longitudinal direction are parallel to each other; and an ultrasound unit built into the insertion section at a position on another end side thereof, in which the housing includes an outer surface, in which the holding section includes: a first axis that matches with the center axis of the insertion section; and a second axis that passes through a center of a cross section taken in the vicinity of a longitudinal center of the housing, and extends in parallel to the longitudinal direction, in which, in a cross section perpendicular to the longitudinal direction of the holding section, the first axis is offset from the second axis in an offset direction, in which, in the cross section perpendicular to the longitudinal direction of the holding section, the outer surface of the holding section includes: a first outer surface portion located on a line extending from the second axis in the offset direction; and a second outer surface portion located on a line extending from the second axis in a direction opposite to the offset direction, and in which a curvature of the first outer surface portion is smaller than a curvature of the second outer surface portion.

In a preferred embodiment of the present invention, in the cross section perpendicular to the longitudinal direction of the holding section, the first outer surface portion includes a substantial arc, and a center of the substantial arc is located in the vicinity of the first axis in the cross section.

In a preferred embodiment of the present invention, the housing includes a first bulging portion in the vicinity of an end portion of the holding section on the insertion section side in the longitudinal direction, and a width of the first bulging portion in the offset direction in the cross section perpendicular to the longitudinal direction is larger than a width of the holding section in the offset direction in the cross section taken in the vicinity of the longitudinal center.

In a preferred embodiment of the present invention, the housing includes a second bulging portion in the vicinity of an end portion of the holding section on a side opposite to the insertion section side in the longitudinal direction, and a width of the second bulging portion in the offset direction in the cross section perpendicular to the longitudinal direction is larger than the width of the holding section in the cross section taken in the vicinity of the longitudinal center.

In a preferred embodiment of the present invention, the width of the first bulging portion in the offset direction in the cross section is larger than the width of the second bulging portion in the offset direction in the cross section.

In a preferred embodiment of the present invention, the first outer surface portion of the holding section includes a substantially straight line portion.

In a preferred embodiment of the present invention, the outer surface has a substantially symmetrical shape across a plane including the first axis and the second axis.

Further, according to the present invention, there is provided an intracavitary ultrasound probe, including: a housing including: an insertion section having a center axis; and a holding section extending in a longitudinal direction of the housing, the holding section being connected to one end of the insertion section so that the center axis and the longitudinal direction are parallel to each other; and an ultrasound unit built into the insertion section at a position on another end side thereof, in which the housing includes an outer surface, in which a width of a contour of the outer surface in a cross section perpendicular to the longitudinal direction of the holding section is larger in a first direction than in a second direction orthogonal to the first direction, in which the contour in the cross section includes: a first arc centered on a first point that is located on a straight line parallel to the first direction; and a second arc centered on a second point that is located on the straight line, the second arc having a curvature larger than the first arc, and in which, in the cross section, the first point is closer to a point of intersection between the center axis of the insertion section and the cross section with respect to the second point.

In a preferred embodiment of the present invention, the housing includes a first bulging portion in the vicinity of an end portion of the holding section on the insertion section side in the longitudinal direction, and a width of the first bulging portion in the first direction in the cross section is larger than a width of the holding section in the first direction in the cross section taken in the vicinity of the longitudinal center.

In a preferred embodiment of the present invention, the housing includes a second bulging portion in the vicinity of an end portion of the holding section on a side opposite to the insertion section side in the longitudinal direction, and a width of the second bulging portion in the first direction in the cross section is larger than a width of the holding section in the first direction in the cross section taken in the vicinity of the longitudinal center.

In a preferred embodiment of the present invention, the width of the first bulging portion in the first direction in the cross section is larger than the width of the second bulging portion in the first direction in the cross section.

In a preferred embodiment of the present invention, the contour in the cross section of the holding section includes a substantially straight line portion located opposite to the second point across the first point in the first direction.

In a preferred embodiment of the present invention, the contour in the cross section has a symmetrical shape across the straight line parallel to the first direction.

In a preferred embodiment of the present invention, a distance between the first point and the second point is larger than a difference between a radius of the first arc and a radius of the second arc.

### ADVANTAGEOUS EFFECTS OF INVENTION

According to the intracavitary ultrasound probe of the present invention, the curvature of the first outer surface portion of the holding section is smaller than the curvature of the second outer surface portion thereof. Therefore, the intracavitary ultrasound probe can be supported stably.

### BRIEF DESCRIPTION OF DRAWINGS

FIG. **1** is a side view of an intracavitary ultrasound probe according to an embodiment of the present invention.
FIG. **2** is a top view of the intracavitary ultrasound probe of FIG. **1****.**
FIG. **3** is a front view of the intracavitary ultrasound probe of FIG. **1****.**
FIG. **4** is a rear view of the intracavitary ultrasound probe of FIG. **1****.**
FIG. **5** is a sectional view of the intracavitary ultrasound probe of FIG. **1** that is taken along the line A-A of FIG. **1****.**
FIG. **6** is a sectional view of the intracavitary ultrasound probe of FIG. **1** that is taken along the line B-B of FIG. **1****.**
FIG. **7** is a sectional view of the intracavitary ultrasound probe of FIG. **1** that is taken along the line C-C of FIG. **1****.**
FIG. **8** is a sectional view of the intracavitary ultrasound probe of FIG. **1** that is taken along the line D-D of FIG. **1****.**
FIG. **9** is a sectional view of the intracavitary ultrasound probe of FIG. **1** that is taken along the line E-E of FIG. **1****.**
FIG. **10** is a sectional view of the intracavitary ultrasound probe of FIG. **1** that is taken along the line F-F of FIG. **1****.**
FIG. **11** is a view illustrating a state of the intracavitary ultrasound probe of FIG. **1** in use.
FIG. **12** is a view illustrating another state of the intracavitary ultrasound probe of FIG. **1** in use.

### DESCRIPTION OF EMBODIMENTS

The inventors of the present application have conducted a detailed study on the operability of the conventional intracavitary ultrasound probe. As a result, the inventors of the present application have found that the holding section of the conventional intracavitary ultrasound probe has a circular cross section, and hence the holding section is hard to rotate with fingers so that the intracavitary ultrasound probe rotates about an axis extending along a longitudinal direction of the intracavitary ultrasound probe.

Therefore, an operator such as a doctor is liable to rotate the intracavitary ultrasound probe by moving his/her wrist joint. In this case, however, the axis extending along the longitudinal direction of the intracavitary ultrasound probe is shifted, with the result that the insertion section turns adversely.

In consideration of those matters, the inventors of the present application have arrived at an intracavitary ultrasound probe having excellent operability. In the following, an intracavitary ultrasound probe according to an embodiment of the present invention is described in detail.

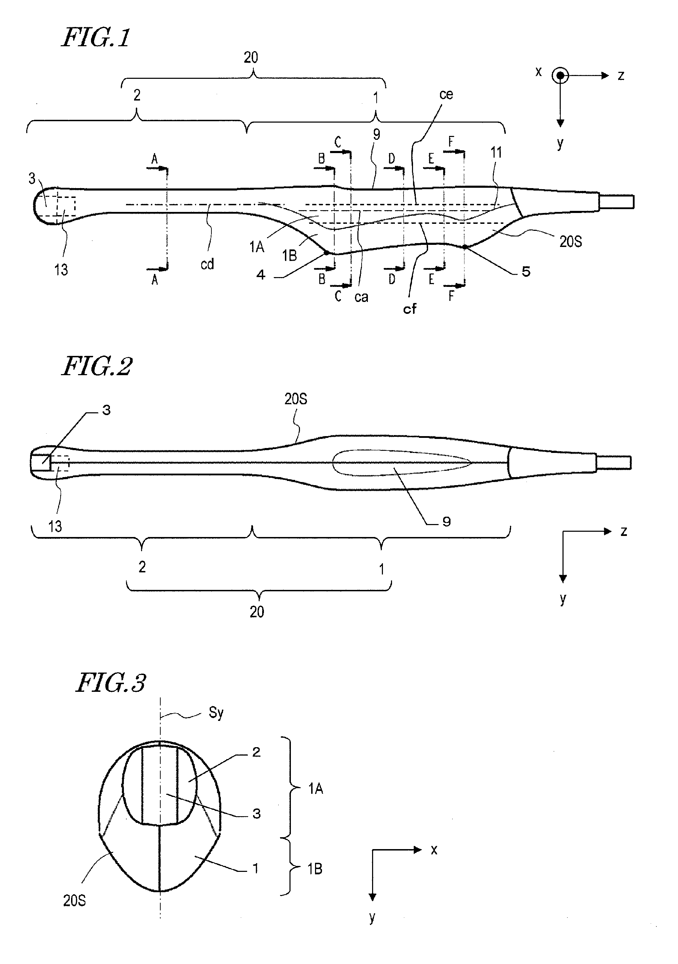

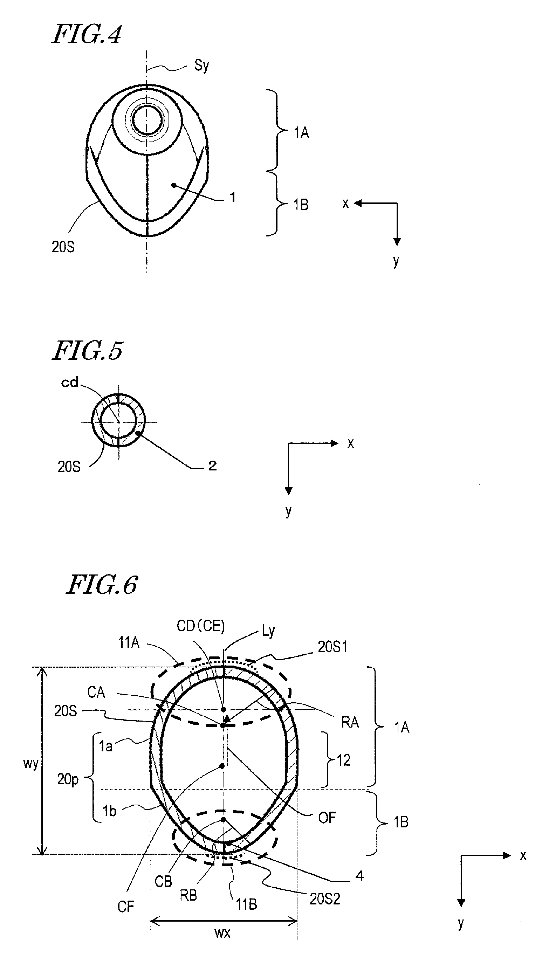

FIG. **1** is a side view of the intracavitary ultrasound probe according to the embodiment of the present invention. FIG. **2** is a top view of the intracavitary ultrasound probe. FIG. **3** and FIG. **4** are a front view and a rear view of the intracavitary ultrasound probe, respectively. As illustrated in those figures, a z-axis is defined as a longitudinal direction of the intracavitary ultrasound probe, and an x-axis and a y-axis are defined in a plane perpendicular to the longitudinal direction. In the following, the intracavitary ultrasound probe is described with reference to those axes as necessary.

As illustrated in FIG. **1****,** the intracavitary ultrasound probe includes a housing **20** having an outer surface **20S,** and an ultrasound unit **13.** As illustrated in FIGS. **1** and **2****,** the housing **20** includes an insertion section **2** and a holding section **1.** The insertion section **2** is formed into a rod shape having a center axis **cd** extending along the longitudinal direction (z-axis), and one end of the insertion section **2** is connected to one end of the holding section **1** so that the longitudinal direction of the holding section **1** is parallel to the center axis **cd.** For example, the housing **20** is made of a resin, and the insertion section **2** and the holding section **1** may be formed by molding integrally with each other.

The ultrasound unit **13** is built into the insertion section **2** at a position on another end side thereof. An acoustic lens **3** is formed on an outer surface of the ultrasound unit **13.** The ultrasound unit **13** is configured to transmit and receive an ultrasonic wave via the acoustic lens **3.** As the ultrasound unit **13** and the acoustic lens **3,** there may be used a publicly known ultrasound unit and acoustic lens which are used for the ultrasound probe.

FIGS. **5** to **10** illustrate cross sections at positions indicated by the lines A-A to F-F of FIG. **1****,** respectively. In FIGS. **5** to **10****,** the housing **20** has a hollow shape with a substantially uniform thickness. However, the housing **20** does not need to have a uniform thickness or a hollow shape.

As illustrated in FIG. **5****,** the insertion section **2** of this embodiment has a circular cross section at a center portion in the longitudinal direction, and the outer surface **20S** has a circular contour in the insertion section **2.** The center axis **cd** matches with a center of the circle defined by the contour. The insertion section **2** does not need to have a circular cross section. In this embodiment, the other end of the insertion section **2** provided with the ultrasound unit **13** has an arc-like contour in the side view of FIG. **1****.** Further, as illustrated in FIGS. **1, 2,** and **3****,** at a portion provided with the ultrasound unit **13,** the insertion section **2** has a larger outer shape.

As illustrated in FIGS. **1** to **4** and **6** to **10,** the entire holding section **1** has a cylindrical shape or a columnar shape extending in the longitudinal direction. More specifically, the holding section **1** is formed of an upper substantially U-shaped member **1A** and a lower substantially U-shaped member **1B,** and the entire holding section **1** has a cylindrical shape or a columnar shape extending in the longitudinal direction. Therefore, the outer surface **20S** has a substantially U-shaped contour **1a** and a substantially U-shaped contour **1b** in an arbitrary cross section taken along a longitudinal direction of the upper substantially U-shaped member **1A** and the lower substantially U-shaped member **1B,** respectively. Further, as illustrated in FIG. **1****,** the holding section **1** has a first axis **ce** that matches with the center axis **ca** of the insertion section **2,** and a second axis **cf** that passes through a center of the cross section taken in the vicinity of the longitudinal center of the holding section **1,** and extends in parallel to the longitudinal direction.

In this embodiment, the U-shape refers to such a shape that ends of two substantially straight line portions on one side are connected to each other through a line including at least an arc portion. The substantially straight line portions may be substantially parallel to each other, or ends of the two substantially straight line portions on another side may be spaced farther away from each other than the ends on one side, to thereby define a V-shape. That is, it is only necessary that the line connecting the two substantially straight line portions include the arc portion. The line portion connecting the two substantially straight line portions is referred to as "bottom portion".

As illustrated in FIGS. **6** to **10****,** the upper substantially U-shaped member **1A** and the lower substantially U-shaped member **1B** have U-shaped openings joined to each other, to thereby define an enclosed space. Therefore, the contour **1a** and the contour **1b** also define an enclosed contour **20p.**

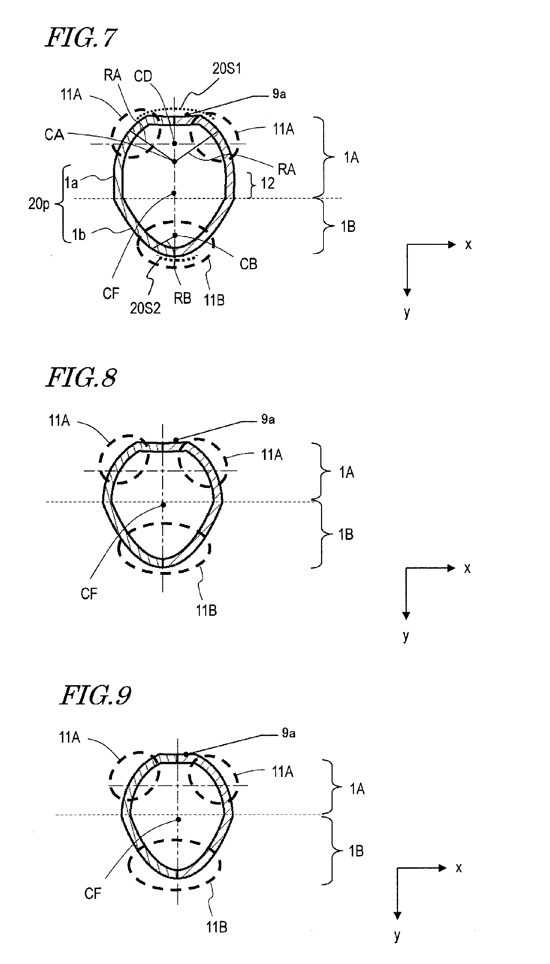

In the cross section perpendicular to the longitudinal direction of the holding section **1,** the outer surface **20S,** that is, the contour **20p,** has a width **wy** in the y-axis direction, and a width **wx** in the x-axis direction. The width is smaller than the width **wy.** In other words, the width **wy** is larger than the width **wx.** Although the illustration is omitted in FIGS. **7** to **10** for clear understanding, it is preferred that, in an arbitrary cross section perpendicular to the longitudinal direction of the holding section **1,** the width **wy** is larger than the width **wx.**

As illustrated in FIG. **6****,** in the cross section perpendicular to the longitudinal direction of the holding section **1,** the contour **1a** and the contour **1b** have a first arc **11A** and a second arc **11B,** respectively. The first arc **11A** is a part of a circle having a radius **RA** about a first point **CA** on a straight line **Ly** parallel to the y-axis, and the second arc **11B** is a part of a circle having a radius **RB** about a second point **CB** on the straight line **Ly.** The radius **RA** is larger than the radius **RB.** Therefore, a curvature (1/RB) of the second arc **11B** is larger than a curvature (1/RA) of the first arc **11A.**

The first point **CA** is closer to a point **CD** with respect to the second point **CB.** The point **CD** is a point at which the center axis **cd** of the insertion section **2** intersects the cross section perpendicular to the longitudinal direction of the holding section **1.** Further, a distance between the first point **CA** and the second point **CB** is larger than a difference between the radius of the first arc **11A** and the radius of the second arc **11B.**

As illustrated in FIG. **6****,** a point **CE** is defined as a point at which the cross section perpendicular to the longitudinal direction of the holding section **1** intersects the first axis **ce,** and a point **CF** is defined as a point at which the cross section perpendicular to the longitudinal direction of the holding section **1** intersects the second axis **cf.** Then, the point **CE** matches with the point **CD.** In this cross section, the point **CE** of the first axis **ce** is offset from the point **CF** of the second axis **cf** in a minus direction of the y-axis. This direction is referred to as "offset direction **OF".** Further, in the cross section perpendicular to the longitudinal direction of the holding section **1,** a part of the outer surface **20S** which is located on the straight line **Ly** extending from the point **CE** in the offset direction **OF** is defined as a first outer surface portion **20S1,** and a part of the outer surface **20S** which is located on the straight line **Ly** extending from the point **CF** in a direction opposite to the offset direction is defined as a second outer surface portion **20S2.** Considering the above-mentioned feature in terms of the straight line **Ly,** it can be said that the first arc **11A** and the second arc **11B** include the first outer surface portion **20S1** and the second outer surface portion **20S2** on the straight line **Ly,** respectively, and that a curvature of the first outer surface portion **20S1** is smaller than a curvature of the second outer surface portion **20S2.** Further, the point **CA** as the center of the first arc **11A** is closer to the point **CE.**

In this embodiment, in arbitrary cross sections perpendicular to the longitudinal direction of the holding section **1,** the positions of the first point **CA** are substantially the same. Therefore, as illustrated in FIG. **1****,** the first point **CA** in an arbitrary cross section is located on an axis **ca.** That is, in the upper substantially U-shaped member **1A,** the shape and position of the curved surface defined by the first arc **11A** are substantially uniform in the longitudinal direction. On the other hand, as illustrated in FIGS. **6** to **10****,** in arbitrary cross sections perpendicular to the longitudinal direction of the holding section **1,** the positions of the second point **CB** are changed. This is because, as described below, a first bulging portion **4** and a second bulging portion **5** are formed in the lower substantially U-shaped member **1B.**

As illustrated in FIGS. **1** and **2****,** the contour **20p** of this embodiment has a symmetrical shape across the straight line **Ly** in the cross section perpendicular to the longitudinal direction of the holding section **1.** Further, the holding section **1** includes a flat surface portion **9** extending in the longitudinal direction at a part of the upper substantially U-shaped member **1A** which is located at the bottom portion having the U-shape. With the flat portion **9,** as illustrated in FIGS. **7** to **10****,** the contour **20p** includes a straight line portion **9a** in the cross section perpendicular to the longitudinal direction of the holding section **1.** The straight line portion **9a** is located at the bottom portion of the contour **1a** having the U-shape, that is, located opposite to the second point **CB** across the first point **CA** in the y-axis direction. The straight line portion **9a** has a substantially straight line shape. Accordingly, as illustrated in FIGS. **7** to **10****,** at the position ranging from the C-C cross section to the F-F cross section, the first arc **11A** is divided into two parts by the straight line portion **9a.** Also in this case, the curvature of the straight line portion **9a** is zero or close to zero, and the first arc **11A** has a smaller curvature than the second arc **11B.** Thus, it can be said that the curvature of the first outer surface portion **20S1** is smaller than the curvature of the second outer surface portion **20S2** even when both the curvature of the straight line portion **9a** and the curvature of the first arc **11A** are compared as the curvature of the first outer surface portion **20S1** to the curvature of the second arc **11B** as the curvature of the second outer surface portion **20S2.**

Further, as illustrated in FIGS. **1** and **6** to **10,** the housing **20** includes the first bulging portion **4** in the vicinity of the end portion of the holding section **1** on the insertion section **2** side in the longitudinal direction. As is apparent through comparison between FIG. **6** and FIG. **8****,** the width **wy** in the y-direction on the cross section on which the first bulging portion **4** is located (FIG. **6****)** is larger than the width **wy** in the y-direction on the cross section taken in the vicinity of the longitudinal center of the holding section **1** (FIG. **8****).** This is because, in this embodiment, the contour **1a** includes an enlarged portion **12** in the first bulging portion **4** and therefore the depth of the U-shape is increased. The enlarged portion **12** may be provided to the contour **1b.**

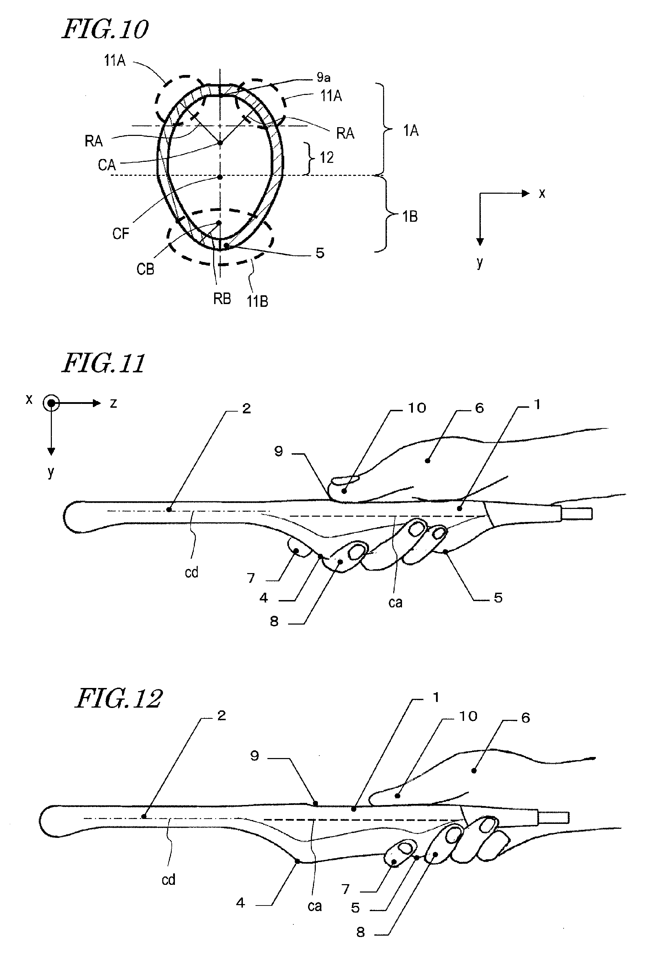

Similarly, the housing **20** includes the second bulging portion **5** in the vicinity of the end portion of the holding section **1** on a side opposite to the insertion section **2** in the longitudinal direction. As is apparent through comparison between FIG. **8** and FIG. **10****,** the width **wy** in the y-direction on the cross section on which the second bulging portion **5** is located (FIG. **10****)** is larger than the width **wy** in the y-direction on the cross section taken in the vicinity of the longitudinal center of the holding section **1** (FIG. **8****).** The width **wy** in the y-axis direction at the first bulging portion **4** is larger than the width **wy** in the y-axis direction at the second bulging portion **5.**

FIG. **11** illustrates a state before inserting the insertion section **2** into a body cavity by holding the intracavitary ultrasound probe of this embodiment by hand. An operator such as a doctor mainly grips the holding section **1** of the housing **20** by hand to hold the intracavitary ultrasound probe. Specifically, for example, the operator puts his/her index finger **7** on the lower substantially U-shaped member **1B** at a position on the insertion section **2** side of the first bulging portion **4,** and puts his/her middle finger **8** on the lower substantially U-shaped member **1B** at a position on the rear side of the first bulging portion **4.** Further, the operator puts his/her ring finger and little finger on the lower substantially U-shaped member **1B** at a position between the first bulging portion **4** and the second bulging portion **5** of the holding section **1.**

The operator puts his/her thumb **10** and a part of his/her palm at the base of the thumb **10** on the flat surface portion **9** of the upper substantially U-shaped member **1A** of the holding section **1.** The palm mainly comes into contact with the half of the upper substantially U-shaped member **1A**. As illustrated in FIG. **6****,** in the cross section in the longitudinal direction, the contour of the holding section **1** has a larger width in the y-axis direction than in the x-axis direction. Therefore, the bottom portion of the lower substantially U-shaped member **1B** of the holding section **1** is easily supported at the distal ends of the fingers, in particular, in the vicinity of the distal end portions of the index finger **7** and the middle finger **8.** Further, the second arc located at the bottom portion of the lower substantially U-shaped member **1B** has a large curvature (small radius), and hence the bending fingers are easily fitted with the lower substantially U-shaped member **1B**. On the other hand, the first arc located at the bottom portion of the upper substantially U-shaped member **1A** has a small curvature (large radius), and hence the area of contact between the palm and the upper substantially U-shaped member **1A** becomes larger. Thus, the intracavitary ultrasound probe can stably be supported at the upper substantially U-shaped member **1A** of the holding section **1.**

In this state, the operator inserts the insertion section **2** into the body cavity and then rotates the insertion section **2** inside the body cavity about the center axis **cd** of the insertion section **2.** With this operation, an ultrasonic wave can be transmitted from the ultrasound unit **13** in arbitrary directions, and hence a reception signal can be obtained from a wide range of the body cavity.

At this time, the operator rotates the holding section **1** of the intracavitary ultrasound probe by his/her hand holding the holding section **1** of the intracavitary ultrasound probe, to thereby rotate the insertion section **2.** The holding section **1** can be rotated at three points of, for example, the thumb **10,** the index finger **7,** and the middle finger **8.** As described above, in the cross section in the longitudinal direction, the contour of the holding section **1** has a larger width in the y-axis direction than in the x-axis direction. Therefore, when a fulcrum is defined on the longitudinal axis in the vicinity of the thumb **10** put on the flat surface portion **9** located at the bottom portion of the upper substantially U-shaped member **1A**, and a power point is defined on the bottom portion of the lower substantially U-shaped member **1B** on which the index finger **7** and the middle finger **8** are put, a larger distance is secured from the fulcrum to the power point. Thus, based on the principle of leverage, the bottom portion of the lower substantially U-shaped member **1B** is lightly moved in the x-axis direction with the index finger **7** and the middle finger **8,** and accordingly the holding section **1** can be rotated about the axis extending along the longitudinal direction, with the result that satisfactory rotation operability can be obtained.

At this time, the radius of the first arc of the upper substantially U-shaped member **1A** is large, and hence a part of the upper substantially U-shaped member **1A** may come into contact with the palm. Further, the feeling of rotation of the upper substantially U-shaped member **1A** substantially dominates the feeling of rotation of the holding section **1.** Therefore, the intracavitary ultrasound probe can be rotated in a manner of sliding in contact with the palm at the part of the upper substantially U-shaped member **1A.** Accordingly, the holding section **1** is easy to rotate about the axis **ca** on which the center of the first arc of the upper substantially U-shaped member **1A** is located. The center of the first arc is located in the vicinity of the center axis **cd** of the insertion section **2,** and hence, as described above, through the rotation of the holding section **1,** the insertion section **2** can be rotated substantially about the center axis **cd** thereof. Thus, the rotation axis of the holding section **1** substantially matches with the rotation axis of the insertion section **2,** and hence the feeling of rotation of the holding section **1** substantially matches with the feeling of rotation of the insertion section **2,** with the result that the operator can obtain an excellent feeling of operation.

FIG. **12** illustrates a case of operating the intracavitary ultrasound probe under a state in which the operator holds the rear end side of the holding section **1.** Also in this case, for example, the operator may put his/her thumb **10** on the flat surface portion **9,** put his/her index finger **7** on the distal end side of the second bulging portion **5,** and put his/her middle finger **8** on the rear end side of the second bulging portion **5.**

Thus, similarly to the holding state illustrated in FIG. **11****,** the intracavitary ultrasound probe can be operated at three points of the thumb **10,** the index finger **7,** and the middle finger **8,** with the result that the operator can obtain a stable feeling of operation and satisfactory rotation operability. Further, the feeling of rotation of the holding section **1** matches with the feeling of rotation of the insertion section **2,** with the result that the operator can obtain an excellent feeling of operation.

Note that, in this embodiment, the upper substantially U-shaped member **1A** and the lower substantially U-shaped member **1B** have the U-shaped openings joined to each other, to thereby define the enclosed space. However, the structure of the housing is not necessarily limited to the structure of the housing in which the upper and lower portions are separated from each other. It is only necessary that the outer surface have the above-mentioned shape, and as long as the enclosed space is defined, the housing may have a structure in which a C-shaped member and an inverted C-shaped member are joined to each other side by side. Alternatively, the housing may have a structure with a rectangular cylindrical shape.

### INDUSTRIAL APPLICABILITY

The present invention is suitably applied to an intracavitary ultrasound probe to be used for various diagnoses. In particular, the present invention is suitably applied to an intracavitary ultrasound probe to be used, for example, to check a growth state of a fetus and a uterine fibroid state.

### REFERENCE SIGNS LIST

- **1**: holding section
- **1A**: upper substantially U-shaped member
- **1B**: lower substantially U-shaped member
- **1a, 1b, 20p**: contour
- **2**: insertion section
- **3**: acoustic lens
- **4**: first bulging portion
- **5**: second bulging portion
- **6**: hand
- **7**: index finger
- **8**: middle finger
- **9**: flat surface portion
- **10**: thumb
- **20**: housing
- **20S**: outer surface

## Claims

1. An intracavitary ultrasound probe, comprising:
a housing comprising:
an insertion section having a center axis; and
a holding section extending in a longitudinal direction of the housing,
the holding section being connected to one end of the insertion section so that the center axis and the longitudinal direction are parallel to each other; and
an ultrasound unit built into the insertion section at a position on another end side thereof,
wherein the housing comprises an outer surface,
wherein the holding section comprises:
a first axis that matches with the center axis of the insertion section; and
a second axis that passes through a center of a cross section taken in the vicinity of a longitudinal center of the housing, and extends in parallel to the longitudinal direction,
wherein, in a cross section perpendicular to the longitudinal direction of the holding section, the first axis is offset from the second axis in an offset direction,
wherein, in the cross section perpendicular to the longitudinal direction of the holding section, the outer surface of the holding section comprises:
a first outer surface portion located on a line extending from the second axis in the offset direction; and
a second outer surface portion located on a line extending from the second axis in a direction opposite to the offset direction, and
wherein a curvature of the first outer surface portion is smaller than a curvature of the second outer surface portion.

2. The intracavitary ultrasound probe according to claim 1,
wherein, in the cross section perpendicular to the longitudinal direction of the holding section, the first outer surface portion comprises a substantial arc, and
wherein a center of the substantial arc is located in the vicinity of the first axis in the cross section.

3. The intracavitary ultrasound probe according to claim 2,
wherein the housing comprises a first bulging portion in the vicinity of an end portion of the holding section on the insertion section side in the longitudinal direction, and
wherein a width of the first bulging portion in the offset direction in the cross section perpendicular to the longitudinal direction is larger than a width of the holding section in the offset direction in the cross section taken in the vicinity of the longitudinal center.

4. The intracavitary ultrasound probe according to claim 3,
wherein the housing comprises a second bulging portion in the vicinity of an end portion of the holding section on a side opposite to the insertion section side in the longitudinal direction, and
wherein a width of the second bulging portion in the offset direction in the cross section perpendicular to the longitudinal direction is larger than the width of the holding section in the cross section taken in the vicinity of the longitudinal center.

5. The intracavitary ultrasound probe according to claim 4, wherein the width of the first bulging portion in the offset direction in the cross section is larger than the width of the second bulging portion in the offset direction in the cross section.

6. The intracavitary ultrasound probe according to any one of claims 1 to 5, wherein the first outer surface portion of the holding section comprises a substantially straight line portion.

7. The intracavitary ultrasound probe according to any one of claims 1 to 6, wherein the outer surface has a substantially symmetrical shape across a plane including the first axis and the second axis.
